# EUROPEAN PATENT SPECIFICATION

(11) **EP 3 858 250 B1**
(45) Date of publication and mention of the grant of the patent: **05.02.2025**
(21) Application number: 20217064.3
(22) Date of filing: 23.12.2020
(51) Int. Cl.: A61B 8/08, A61B 8/06

(54) **ULTRASOUND IMAGING APPARATUS AND OPERATION METHOD THEREOF**
ULTRASCHALLBILDGEBUNGSVORRICHTUNG UND BETRIEBSVERFAHREN DAFÜR
APPAREIL D'IMAGERIE À ULTRASONS ET PROCÉDÉ DE FONCTIONNEMENT ASSOCIÉ

(30) Priority: 29.01.2020 KR 20200010486
(43) Date of publication of application: 04.08.2021
(73) Proprietor: Samsung Medison Co., Ltd., Hongcheon-gun, Gangwon-do 25108 (KR)
(72) Inventor: Kim, Beomgyu, 05340 Seoul (KR); Lee, Jaesung, 05340 Seoul (KR); Im, Jiun, 05340 Seoul (KR)
(74) Representative: Arnold & Siedsma

(56) References cited:
- US-A1- 2014 276 072
- US-B1- 6 176 830
- US-B2- 10 342 515

## Description

### BACKGROUND

### 1. Field

The disclosure relates to ultrasound imaging apparatuses and operation methods thereof, and more particularly, to ultrasound imaging apparatuses and operation methods for correcting an angle of a sample volume by taking into account a direction of blood flow in a spectral Doppler mode.

### 2. Description of Related Art

Ultrasound imaging apparatuses transmit, to an object, ultrasound signals generated by transducers of a probe and receive information about signals reflected from the object to obtain at least one image of an internal part (e.g., soft tissue or blood flow) of the object.

In a spectral Doppler mode, quadrature detection is performed on an ultrasound echo signal received via a probe to extract a Doppler signal having a frequency component that undergoes a Doppler shift due to motion of an object such as blood flow or a heart wall. By obtaining a Doppler image from a Doppler signal, a user may identify blood flow information such as a location, a direction, and a velocity of blood flow. In pulse wave (PW) Doppler of the related art, a Doppler signal is generated by obtaining a single receive beam for an ultrasound transmit beam applied to an object via a probe. When the ultrasound transmit beam is not aligned with a direction of blood flow but forms a certain angle with respect thereto, the user needs to manually correct an angle of a sample volume to be aligned with the direction of blood flow in order to accurately measure a velocity of blood flow.

In general, a correction operation for aligning the angle of sample volume with the direction of blood flow is manually performed through a user's naked eye, resulting in low correction accuracy. Furthermore, each time additional imaging is performed or each time the direction of blood flow is changed due to a patient's breathing, the user has to repeatedly perform correction operations to align the angle of the sample volume with the direction of blood flow. Frequent corrections to the angle of the sample volume causes user inconvenience and degrades accuracy of blood velocity measurement. In particular, when there is an error in correcting an angle between directions of the sample volume and blood flow, misdiagnosis may occur.

US 10 243 515 B2 and US 2014/276072 A1 describe correcting the longitudinal angle of the sample volume based on the moving direction of the blood flow, but do not disclose obtaining the trend line, and correcting the angle based on a trend line to acquire the blood flow velocity based on the more accurate moving direction of the blood flow. US 6 176 830 B1 describes angle correction based on the direction of blood flow, but does not describe obtaining a more accurate blood flow rate by acquiring a trend line and correcting the speed (direction) of blood flow using the angle formed by a trend line and the scan line.

### SUMMARY

In accordance with an aspect of the disclosure, a method according to claim 1 is provided.

In accordance with another aspect of the disclosure, an ultrasound imaging apparatus according to claim 6

In accordance with another aspect of the disclosure, a computer-readable recording medium according to claim 11 is provided.

### BRIEF DESCRIPTION OF THE DRAWINGS

The above and other aspects, features, and advantages of certain embodiments of the disclosure will be more apparent from the following description taken in conjunction with the accompanying drawings, in which:
FIG. 1 is a block diagram of a configuration of an ultrasound imaging apparatus according to an embodiment of the disclosure;
FIG. 2 is a flowchart of an operation method of an ultrasound imaging apparatus, according to an embodiment of the disclosure;
FIG. 3 is a diagram for describing an embodiment in which an ultrasound imaging apparatus of the disclosure obtains Doppler data by using a multiline receiving technique;
FIG. 4 is flowchart of a method, performed by an ultrasound imaging apparatus, of obtaining Doppler data by using a multiline receiving technique, according to an embodiment of the disclosure;
FIG. 5 is a diagram for describing an embodiment in which an ultrasound imaging apparatus of the disclosure corrects an angle of a sample volume by using information about a location and a direction of blood flow;
FIG. 6 is a flowchart of a method, performed by an ultrasound imaging apparatus, of correcting an angle of a sample volume by using a location and a direction of blood flow, according to an embodiment of the disclosure;
FIG. 7 illustrates an embodiment in which an ultrasound imaging apparatus of the disclosure displays a trend line for blood flow on a brightness (B)-mode ultrasound image;
FIG. 8A illustrates a frequency spectrum obtained by an ultrasound imaging apparatus at pulse repetition frequency (PRF) intervals, according to an embodiment of the disclosure;
FIG. 8B is a diagram for describing a method performed by an ultrasound imaging apparatus, of updating a trend line for blood flow by using power data for spectral Doppler pulse waves obtained based on a predetermined time period, according to an embodiment of the disclosure;
FIG. 9 is a flowchart of a method performed by an ultrasound imaging apparatus, of updating a trend line for blood flow by using power data for spectral Doppler pulse waves obtained during a predetermined time period, according to an embodiment of the disclosure; and
FIGS. 10A through 10C are diagrams illustrating ultrasound imaging apparatuses according to embodiments of the disclosure.

### DETAILED DESCRIPTION

Although the terms used in the disclosure have been described in general terms that are currently used in consideration of the functions referred to in the disclosure, the terms are intended to encompass various other terms depending on the intent of those skilled in the art, precedents, or the emergence of new technology.

Also, some of the terms used herein may be arbitrarily chosen by the applicant. In this case, these terms are defined in detail below.

Accordingly, the terms used in the disclosure are not defined based on the meaning of the term, not on the name of a simple term, but on the contents throughout the disclosure.

An expression used in the singular encompasses the expression of the plural, unless it has a clearly different meaning in the context.

The terms including technical and scientific terms used herein have the same meaning as commonly understood by one of ordinary skill in the art to which the disclosure belongs.

As used herein, the term "and/or" includes any and all combinations of one or more of the associated listed items. Expressions such as "at least one of," when preceding a list of elements, modify the entire list of elements and do not modify the individual elements of the list.

Throughout the disclosure, the expression "at least one of a, b or c" indicates only a, only b, only c, both a and b, both a and c, both b and c, all of a, b, and c, or variations thereof.

Throughout the entirety of the specification of the disclosure, when it is assumed that a certain part includes a certain element, the term 'including' means that a corresponding element may further include other elements unless a specific meaning opposed to the corresponding element is written.

The term used in the embodiments of the disclosure such as "unit" or "module" indicates a unit for processing at least one function or operation, and may be implemented in hardware, software, or in a combination of hardware and software.

According to the situation, the expression "configured to" used herein may be used as, for example, the expression "suitable for", "having the capacity to", "designed to", "adapted to", "made to", or "capable of".

The term "configured to" must not mean only "specifically designed to" in hardware.

Instead, the expression "a device configured to" may mean that the device is "capable of" operating together with another device or other elements.

For example, a "processor configured to (or set to) perform A, B, and C" may mean a dedicated processor (e.g., an embedded processor) for performing a corresponding operation or a generic-purpose processor (e.g., a central processing unit (CPU) or an application processor) which performs corresponding operations by executing one or more software programs which are stored in a memory device.

In the present specification, an "object" may be a human, an animal, or a part of a human or animal. For example, the object may be an organ (e.g., the liver, the heart, the womb, the brain, a breast, or the abdomen), a blood vessel, or a combination thereof. Furthermore, the "object" may be a phantom. The phantom means a material having a density, an effective atomic number, and a volume that are approximately the same as those of an organism. For example, the phantom may be a spherical phantom having properties similar to the human body.

Furthermore, in the present specification, a "user" may be, but is not limited to, a medical expert, such as a medical doctor, a nurse, a medical laboratory technologist, and a technician who repairs a medical apparatus.

Hereinafter, embodiments of the disclosure will be described in detail with reference to the accompanying drawings.

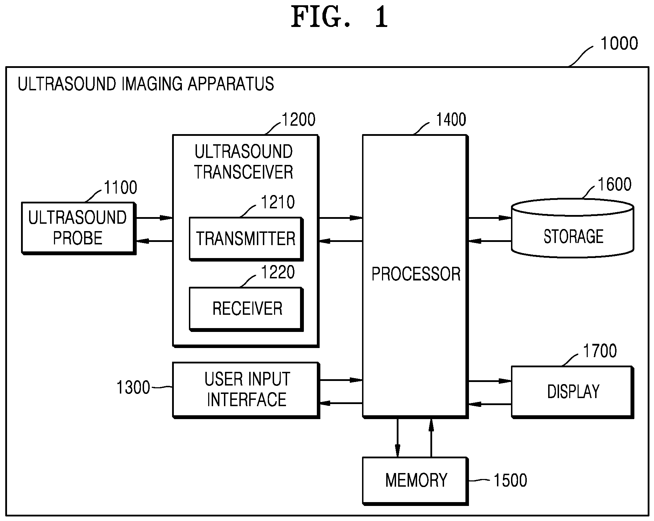

FIG. 1 is a block diagram of a configuration of an ultrasound imaging apparatus 1000 according to an embodiment of the disclosure. The ultrasound imaging apparatus 1000 of FIG. 1 is an apparatus for receiving ultrasound echo signals from an ultrasound probe 1100 and generating an ultrasound image of an object, i.e., an inner part of a patient's body, by performing image processing on the received ultrasound signals. The ultrasound imaging apparatus 1000 may be implemented as a cart-type apparatus, but is not limited thereto. For example, the ultrasound imaging apparatus 1000 may be implemented as a portable-type apparatus including at least one of a picture archiving and communication system (PACS) viewer, a smart phone, a laptop computer, a tablet personal computer (PC), and a personal digital assistant (PDA).

Referring to FIG. 1, the ultrasound imaging apparatus 1000 may include the ultrasound probe 1100, an ultrasound transceiver 1200, a user input interface 1300, a processor 1400, a memory 1500, a storage 1600, and a display 1700. The components shown in FIG. 1 are only according to an embodiment of the disclosure, and components included in the ultrasound imaging apparatus 1000 are not limited to those shown in FIG. 1. The ultrasound imaging apparatus 1000 may not include some of the components shown in FIG. 1 and may further include components not shown in FIG. 1.

The ultrasound probe 1100 may include a plurality of transducer elements that transmit ultrasound signals to an object, i.e., a patient's body, and receive ultrasound echo signals reflected from the patient's body. The ultrasound probe 1100 may be connected to the ultrasound imaging apparatus 1000 by wire or wirelessly. In an embodiment, the ultrasound probe 1100 may be a separate probe that operates independently of the ultrasound imaging apparatus 1000. Furthermore, the ultrasound imaging apparatus 1000 may include one or a plurality of ultrasound probes 1100 according to its implemented configuration.

The transducer elements may transmit ultrasound signals to an object in response to transmit signals applied by a transmitter 1210. The transducer elements may receive ultrasound echo signals reflected from the object to produce receive signals. The processor 1400 may control the transmitter 1210 to produce transmit signals to be applied respectively to the transducer elements considering locations of the transducer elements in the ultrasound probe 1100 and a focal point thereof. According to an embodiment, the transmitter 1210 may produce, according to control by the processor 1400, a transmit signal for controlling the ultrasound probe 1100 to transmit an ultrasound beam once. The processor 1400 may control the receiver 1220 to generate ultrasound data by performing analog-to-digital conversion (ADC) on receive signals provided by the ultrasound probe 1100 and summing the digital receive signals based on the locations and focal point of the transducer elements.

The ultrasound probe 1100 may receive a plurality of ultrasound echo signals along a plurality of scan lines by using a multiline receiving technique. In an embodiment, the ultrasound probe 1100 may transmit an ultrasound beam to a region of interest (ROI) in an object along a single scan line according to control by the transmitter 1210, and a plurality of transducer elements of the ultrasound probe 1100 may receive ultrasound echo signals for the ROI along a plurality of scan lines. When an imaging mode of the ultrasound imaging apparatus 1000 is a spectral Doppler mode, the ultrasound probe 1100 may receive Doppler data at a plurality of points in the ROI along a plurality of scan lines. In this case, the Doppler data may include spectral Doppler pulse wave information.

In an embodiment, the ultrasound probe 1100 may obtain a plurality of pieces of Doppler data by repeatedly receiving ultrasound echo signals reflected from the object a plurality of times at pulse repetition frequency (PRF) intervals.

In an embodiment, the ROI may include a sample volume and may be a region wider than the sample volume. A sample volume refers to a region set to a specific depth value on any one of a plurality of scan lines. In an embodiment, a sample volume may be set based on a user input.

The user input interface 1300 may receive a user input for controlling the ultrasound imaging apparatus 1000. For example, the user input interface 1300 may receive user inputs via a button, a keypad, a mouse, a trackball, a jog switch, a knob, etc., a touch input for touching a touch pad or touch screen, a drag input, a swipe input, a voice input, a motion input, an input of biometric information (e.g., iris recognition, fingerprint recognition, etc.), etc. In an embodiment, to enter a spectral Doppler mode, the user input interface 1300 may receive a user input such as pressing a specific button or touching a graphical user interface (GUI) displayed on the display 1700. In an embodiment, the user input interface 1300 may receive a user input for setting an ROI in a spectral Doppler image or an ultrasound B- mode image.

The processor 1400 may control all operations of the ultrasound imaging apparatus 1000 and flow of signals among components within the ultrasound imaging apparatus 1000. The processor 1400 may execute one or more instructions of a program stored in the memory 1500. The processor 1400 may be composed of hardware components for performing arithmetic, logic and input/output operations, and signal processing. For example, the processor 1400 may be configured with at least one of a central processing unit (CPU), a microprocessor, a graphics processing unit (GPU), application specific integrated circuits (ASICs), digital signal processors (DSPs), digital signal processing devices (DSPDs), programmable logic devices (PLDs), and field programmable gate arrays (FPGAs), but is not limited thereto.

A program including instructions for controlling the ultrasound imaging apparatus 1000 may be stored in the memory 1500. The memory 1500 may store one or more instructions and program code that may be read by the processor 1400. In the following embodiments, the processor 1400 may be implemented by executing code or instructions of a program stored in the memory 1500.

For example, the memory 1500 may include at least one type of storage medium from among random access memory (RAM), static RAM (SRAM), read-only memory (ROM), electrically erasable programmable ROM (EEPROM), PROM, a flash memory-type memory, a hard disk-type memory, a multimedia card micro-type memory, a card-type memory (e.g., an SD card or an XD memory), a magnetic memory, a magnetic disc, and an optical disc.

The processor 1400 may obtain Doppler data for each of a plurality of scan lines by using a multiline receiving technique, obtain information about a location and a direction of blood flow by analyzing power data of blood flow (hereinafter, referred to as blood flow power data) in an ROI based on the Doppler data, and correct an angle of a sample volume based on the obtained information about the location and direction of blood flow.

According to an embodiment, the storage 1600 may store spectral Doppler pulse waves for a plurality of points in an ROI set to have a predetermined size around a position of a sample volume, wherein the spectral Doppler pulse waves are obtained by performing multiline receiving via the ultrasound probe 1100. Spectral Doppler pulse wave information may be classified for each of a plurality of scan lines and stored in the storage 1600. The processor 1400 may obtain data regarding spectral Doppler pulse waves (hereinafter, referred to as spectral Doppler pulse wave data) for each of the scan lines stored in the storage 1600. In an embodiment, the processor 1400 may obtain spectral Doppler pulse wave data for each scan line from the ultrasound probe 1100 and not from the storage 1600.

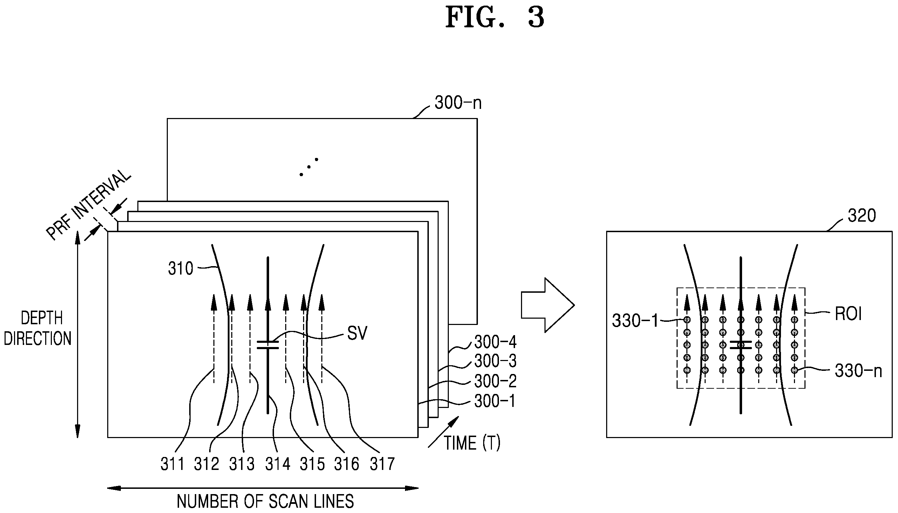

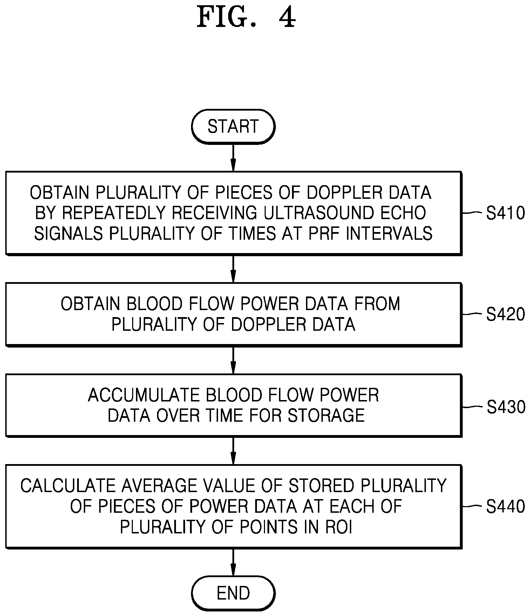

The processor 1400 may obtain blood flow power data from spectral Doppler pulse waves obtained at a plurality of points in an ROI for each of a plurality of scan lines. The processor 1400 may normalize power data values of blood flow based on spectral Doppler pulse wave data obtained at PRF intervals. In an embodiment, the processor 1400 may accumulate, in a time axis direction, N pieces of power data for spectral Doppler pulse waves obtained during a predetermined time period at PRF intervals and calculate an average value of the accumulated power data for each scan line. The processor 1400 may calculate an average value of power data for each of a plurality of points included in each of the scan lines. A detailed method by which the processor 1400 calculates an average value of power data for each of a plurality of points will be described in detail with reference to FIGS. 3 and 4.

The processor 1400 may extract power data having a value greater than or equal to a predetermined threshold from among pieces of blood flow power data. According to an embodiment, the processor 1400 may extract, for each of a plurality of scan lines, a point having an average power data value greater than or equal to a predetermined threshold from among average values of power data calculated at each of a plurality of points.

The processor 1400 may identify points having values of the extracted power data from among a plurality of points in an ROI and obtain a trend line indicating a location and a direction of blood flow. In an embodiment, the processor 1400 may extract power data having a maximum value from among pieces of blood flow power data obtained at a plurality of points for each of a plurality of scan lines and obtain a trend line by connecting points having values of the extracted power data to one another.

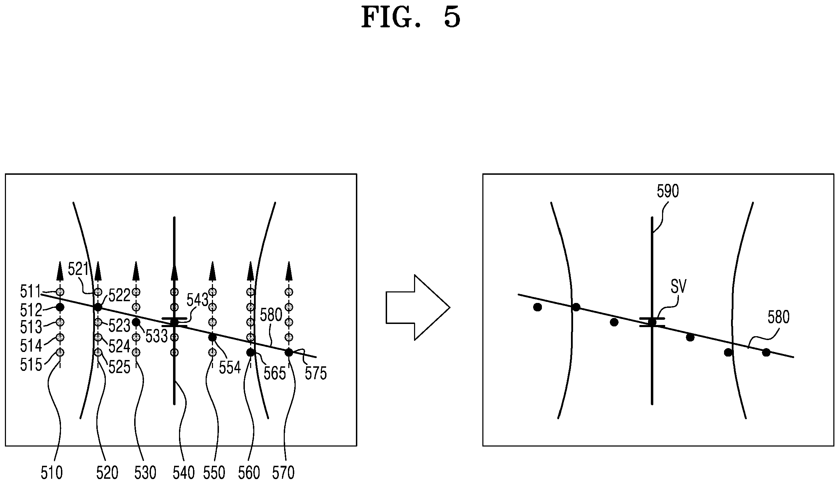

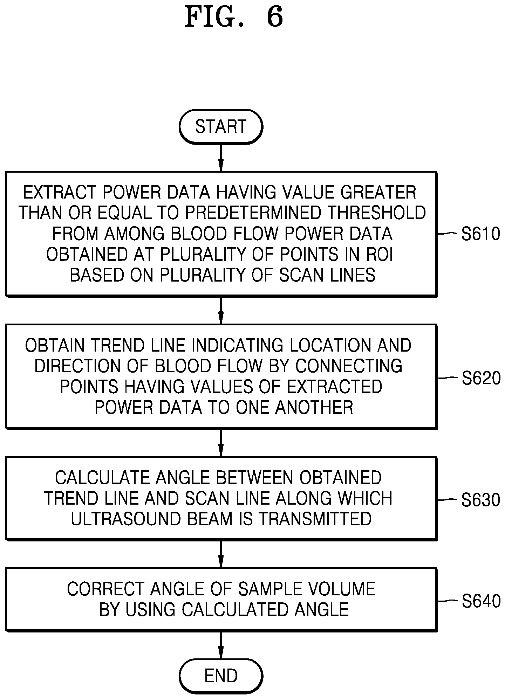

The processor 1400 may calculate an angle between the obtained trend line and a scan line along which an ultrasound beam is transmitted, and correct an angle of a sample volume by using the calculated angle. According to an embodiment, the processor 1400 may calculate an angle θ between a trend line and a scan line on which a sample volume is located from among a plurality of scan lines, and correct an angle by multiplying a velocity of blood flow passing through the sample volume by 1/cos θ. A detailed method by which the processor 1400 obtains a trend line and corrects an angle of a sample volume by using an angle between a trend line and a scan line will be described in detail with reference to FIGS. 5 and 6.

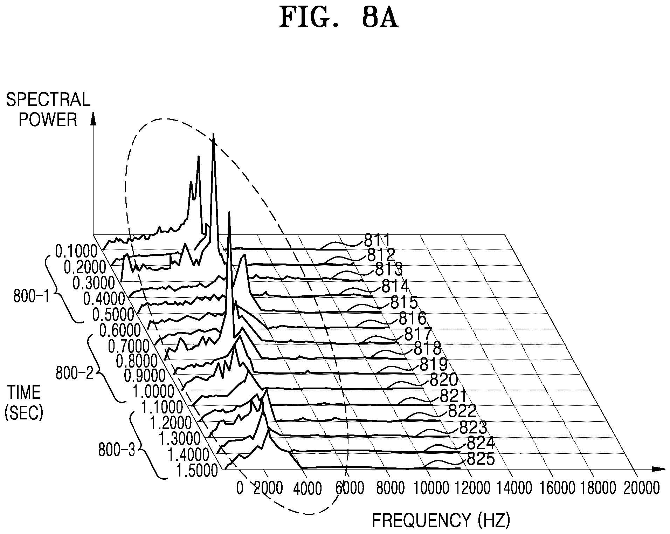

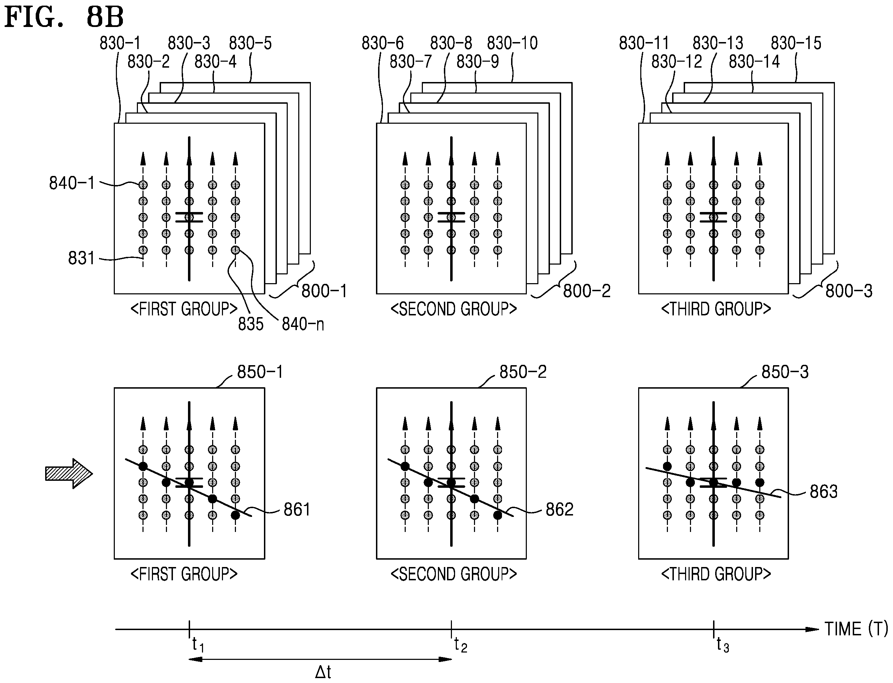

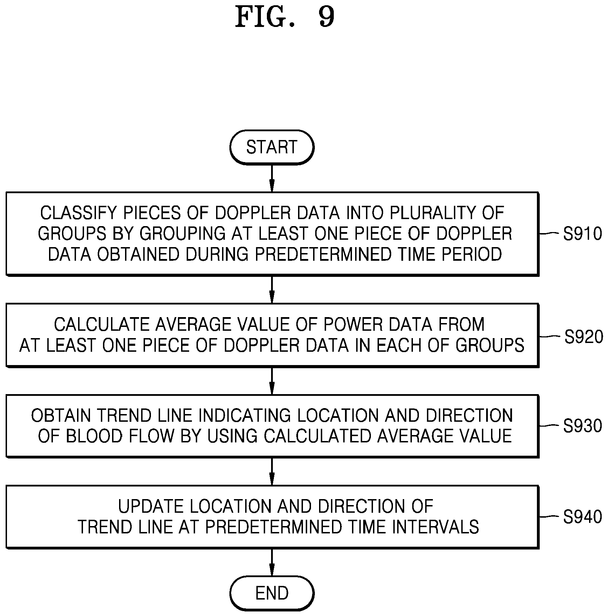

The processor 1400 may update the trend line at predetermined time intervals. In an embodiment, the processor 1400 may classify a plurality of pieces of Doppler data obtained at PRF intervals into a plurality of groups by grouping only at least one piece of Doppler data obtained during a predetermined time period from among the pieces of Doppler data, calculate an average value of power data from the at least one piece of Doppler data included in each of the groups, obtain a trend line by using the calculated average value, and update the obtained trend line based on passage of time. A detailed method by which the processor 1400 updates a trend line based on passage of time will be described in detail below with reference to FIGS. 8A, 8B, and 9.

The processor 1400 may display a trend line on the display 1700. According to an embodiment, the processor 1400 may display a B-mode ultrasound image of the object on the display 1700 and a trend line such that a graphic representing the trend line as a line or image is overlaid on the B-mode ultrasound image. In an embodiment, the processor 1400 may update the trend line displayed on the display 1700 at predetermined time intervals.

Spectral doppler pulse wave information may be classified for each scan line and stored in the storage 1600. The storage 1600 may include at least one type of storage medium from among a flash memory-type memory, a hard disk-type memory, a multimedia card micro-type memory, a card-type memory (e.g., an SD card or an XD memory), a magnetic memory, a magnetic disc, and an optical disc. In an embodiment, the storage 1600 may be implemented in the form of an external database rather than an internal component of the ultrasound imaging apparatus 1000.

For example, the display 1700 may be configured as a physical display including at least one of a cathode-ray tube (CRT) display, a liquid crystal display (LCD), a plasma display panel (PDP), an organic light-emitting diode (OLED) display, a field emission display (FED), a light-emitting diode (LED) display, a vacuum fluorescent display (VFD), a digital light processing (DLP) display, a flat panel display (FPD), a three-dimensional (3D) display, and a transparent display, but is not limited thereto. In an embodiment, the display 1700 may be configured as a touch screen including a touch interface. When the display 1700 is configured as a touch screen, the display 1700 may be a component integrated with the user input interface 1300 formed as a touch panel.

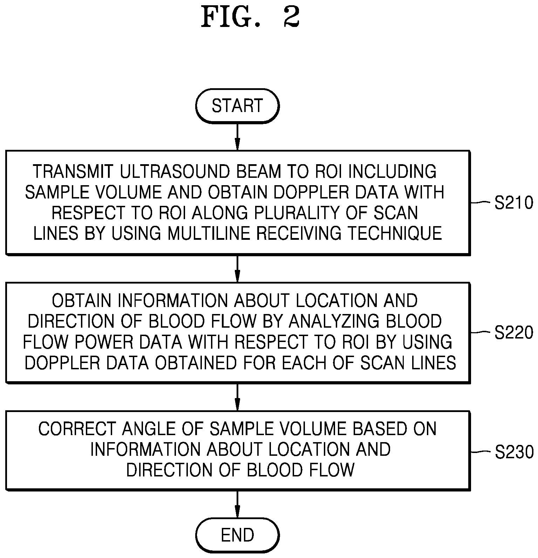

FIG. 2 is a flowchart of an operation method of the ultrasound imaging apparatus 1000, according to an embodiment of the disclosure.

The ultrasound imaging apparatus 1000 transmits an ultrasound beam to an ROI including a sample volume and obtains Doppler data with respect to the ROI based on a plurality of scan lines by using a multiline receiving technique (operation S210). In an embodiment, the ROI may include a sample volume, and may be a region wider than the sample volume. In an embodiment, the ROI may be set based on a user input. According to an embodiment, the ultrasound imaging apparatus 1000 may transmit an ultrasound beam to an ROI via an ultrasound probe and receive ultrasound echo signals for a plurality of points in the ROI along the scan lines. When an imaging mode is set to a spectral Doppler mode, the ultrasound imaging apparatus 1000 may obtain spectral Doppler pulse wave data at a plurality of points in an ROI along a plurality of scan lines by using a multiline receiving technique. According to an embodiment, the ultrasound imaging apparatus 1000 may obtain a plurality of pieces of spectral Doppler pulse wave data over time by repeatedly receiving ultrasound echo signals a plurality of times at PRF intervals.

The ultrasound imaging apparatus 1000 obtains information about a location and a direction of blood flow by analyzing blood flow power data with respect to the ROI based on Doppler data obtained for each of the scan lines (operation S220). According to an embodiment, the ultrasound imaging apparatus 1000 may obtain spectral Doppler pulse wave data at a plurality of points in the ROI for each of the scan lines, and obtain blood flow power data from the spectral Doppler pulse wave data. The ultrasound imaging apparatus 1000 may normalize power data values of blood flow based on spectral Doppler pulse wave data obtained at PRF intervals. In an embodiment, the ultrasound imaging apparatus 1000 may accumulate, in a time axis direction, N pieces of power data for spectral Doppler pulse waves obtained during a predetermined time period at PRF intervals and calculate an average value of the accumulated power data for each of the scan lines. The ultrasound imaging apparatus 1000 may calculate an average value of power data for each of a plurality of points included in each of the scan lines.

The ultrasound imaging apparatus 1000 may extract power data having a value greater than or equal to a predetermined threshold from among blood flow power data. According to an embodiment, the ultrasound imaging apparatus 1000 may extract a point having an average power data value greater than or equal to a predetermined threshold from among average power data values calculated for each of a plurality of points in each of the scan lines. The ultrasound imaging apparatus 1000 may identify points having values of extracted power data from among a plurality of points in an ROI and obtain a trend line indicating a location and a direction of blood flow by connecting the identified points to one another. According to another embodiment, the ultrasound imaging apparatus 1000 may extract power data having a maximum value from among pieces of blood flow power data respectively obtained at a plurality of points for each of the scan lines and obtain a trend line by connecting points having values of the extracted power data to one another.

The ultrasound imaging apparatus corrects an angle of the sample volume based on the information about the location and direction of the blood flow (operation S230). In an embodiment, the ultrasound imaging apparatus 1000 may calculate an angle between a trend line indicating a location and a direction of blood flow and a scan line along which an ultrasound beam is transmitted, and correct an angle of a sample volume by using the calculated angle. According to an embodiment, the ultrasound imaging apparatus 1000 may calculate an angle θ between a trend line and a scan line on which a sample volume is located from among a plurality of scan lines, and correct an angle by multiplying a velocity of the sample volume by 1/cos θ.

According to the related art, to accurately measure a blood flow velocity inside a sample volume in the spectral Doppler mode, the user needs to perform a task of aligning an angle of the sample volume with a direction of the blood flow. To correct the angle of the sample volume, the user has to manually correct the angle of the sample volume with the naked eye. When the location of blood flow changes or the direction of the blood flow changes due to a patient's motion or breathing, the user has to repeatedly perform an operation of correcting the angle of the sample volume. In this case, the user is inconvenienced in having to manipulate the angle of the sample volume each time the location or direction of blood flow changes, and a blood flow velocity cannot be accurately measured by correcting the angle of the sample volume with the naked eye.

According to the embodiments illustrated in FIGS. 1 and 2, the ultrasound imaging apparatus 1000 of the disclosure may obtain spectral Doppler pulse wave data for a plurality of points in an ROI including a sample volume by using a multiline receiving technique, obtain a trend line indicating a location and a direction of blood flow by using the spectral Doppler pulse wave data obtained at the points, and automatically correct an angle of the sample volume based on an angle formed between the trend line and a scan line where the sample volume is located. Accordingly, the ultrasound imaging apparatus 1000 of the disclosure may provide more user convenience by omitting an operation of correcting an angle of a sample volume via a user input. Furthermore, the ultrasound imaging apparatus 1000 of the disclosure may correct the angle of the sample volume by using a trend line indicating a location and a direction of blood flow, thereby increasing the accuracy of measuring a blood flow velocity.

In particular, the ultrasound imaging apparatus 1000 of the disclosure uses spectral Doppler pulse wave data obtained by using the multiline receiving technique in the spectral Doppler mode to correct the angle of the sample volume, thereby eliminating the need to additionally generate a B-mode ultrasound image or color (C)-mode ultrasound image. Thus, a processing time required to generate a B- or C-mode ultrasound image may be reduced, thereby increasing a processing speed and improving a frame rate.

FIG. 3 is a diagram for describing an embodiment in which the ultrasound imaging apparatus 1000 of the disclosure obtains Doppler data by using a multiline receiving technique.

Referring to FIG. 3, the ultrasound imaging apparatus 1000 may obtain a plurality of pieces of Doppler data, i.e., first through n-th pieces of Doppler data 300-1 through 300-n, over time by repeatedly receiving ultrasound echo signals a plurality of times at PRF intervals. In an embodiment, the processor (1400 of FIG. 1) of the ultrasound imaging apparatus 1000 may obtain pieces of Doppler data composed of Doppler-shifted frequency components by performing quadrature detection of ultrasound echo signals, identify Doppler data corresponding to a sample volume SV, transform the identified Doppler data into frequency components, e. g., by performing a fast Fourier transform (FFT) thereon, and obtain spectral Doppler pulse wave data by calculating power of the frequency components.

The ultrasound imaging apparatus 1000 may transmit an ultrasound beam 310 to an ROI via the ultrasound probe (1100 of FIG. 1), and obtain pieces of spectral Doppler pulse wave data at a plurality of points 330-1 through 330-n in the ROI along a plurality of scan lines, i.e., first through seventh scan lines 311 through 317, by using a multiline receiving technique. FIG. 4 shows seven (7) scan lines, i.e., the first through seventh scan lines 311 through 317, for convenience of description, and the number of scan lines (the first through seventh scan lines 311 through 317) is not limited to 7.

The ROI may be a region including the sample volume SV. The sample volume SV may be a region set to a specific depth on the fourth scan line 314 among the first through seventh scan lines 311 through 317. In an embodiment, the sample volume SV may be set based on a user input.

The ultrasound imaging apparatus 1000 may obtain blood flow power data from spectral Doppler pulse wave data obtained at each of the points 330-1 through 330-n, accumulate over time blood flow power data from each of the first through n-th pieces of Doppler data 300-1 through 300-n obtained at PRF intervals, and store the accumulated blood flow power data in the storage (1600 of FIG. 1). The points 330-1 through 330-n refer to points having different depths along the first through seventh scan lines 311 through 317. According to an embodiment, the processor 1400 of the ultrasound imaging apparatus 1000 may calculate a total power data value by adding power data values of blood flow, corresponding to spectral Doppler data obtained at a plurality of points having different depths for each of the first through seventh scan lines 311 through 317 in the first piece of Doppler data 300-1, to power data values of blood flow, corresponding to spectral Doppler data obtained at the points 330-1 through 330-n for each of the first through seventh scan lines 311 through 317 in the second piece of Doppler data 300-2. Similarly, the processor 1400 may accumulatively add power data values of blood flow, corresponding to spectral Doppler data obtained at the points 330-1 through 330-n for each of the first through seventh scan lines 311 through 317 in the third piece of Doppler data 300-3, to a total power data value calculated for each of the points 330-1 through 330-n .

The ultrasound imaging apparatus 1000 may normalize power data values accumulated for each of the points 330-1 through 330-n. In an embodiment, the processor 1400 may calculate an average value of power data for each of the points 330-1 through 330-n by performing an operation of dividing a sum of power data accumulated for each of the points 330-1 through 330-n along each of the first through seventh scan lines 311 through 317 by the number of pieces of Doppler data (the first through n-th pieces of data 300-1 through 300-n) each obtained during a predetermined time period. In an embodiment, the processor 1400 may calculate an average value of power data for each of the points 330-1 through 330-n in the ROI.

FIG. 4 is flowchart of a method performed by the ultrasound imaging apparatus 1000, of obtaining Doppler data by using a multiline receiving technique, according to an embodiment of the disclosure.

The ultrasound imaging apparatus 1000 obtains a plurality of pieces of Doppler data by repeatedly receiving ultrasound echo signals reflected from an object a plurality of times at PRF intervals (operation S410). According to an embodiment, the ultrasound imaging apparatus 1000 may obtain a plurality of pieces of Doppler data for a plurality of points in an ROI based on a plurality of scan lines by using a multiline receiving technique.

The ultrasound imaging apparatus 1000 obtains blood flow power data from the pieces of Doppler data (operation S420). According to an embodiment, the ultrasound imaging apparatus 1000 may perform an FFT to transform the pieces of Doppler data obtained at the points in the ROI into frequency signal components, calculate powers of the frequency signal components, and obtain spectral Doppler pulse wave data by arranging the powers of the frequency signal components along a frequency axis. In an embodiment, the ultrasound imaging apparatus 1000 may obtain blood flow power data from spectral Doppler pulse wave data.

The ultrasound imaging apparatus 1000 accumulates and stores the obtained blood flow power data over time (operation S430). In an embodiment, the ultrasound imaging apparatus 1000 may accumulatively add , power data values obtained at each of a plurality of points in an ROI and store the sum of the power data values calculated for each of the points in the storage 1600 According to an embodiment, a plurality of points refer to points having different depths along a plurality of scan lines. In an embodiment, the ultrasound imaging apparatus 1000 may calculate a sum of power data at each of the points by summing power data values obtained at each of the points having different depths for each of the plurality of scan lines.

The ultrasound imaging apparatus 1000 calculates an average value of stored plurality of pieces of power data at each of a plurality of points in an ROI (operation S440). According to an embodiment, the ultrasound imaging apparatus 1000 may calculate an average power data value for each of the points by performing an operation of dividing a sum of power data values accumulated for each of the points in each of a plurality of scan lines by the number of pieces of Doppler data each obtained during a predetermined time period. In an embodiment, the ultrasound imaging apparatus 1000 may calculate an average value of power data for each of the points in the ROI.

FIG. 5 is a diagram for describing an embodiment in which the ultrasound imaging apparatus 1000 of the disclosure corrects an angle of a sample volume by using information about a location and a direction of blood flow.

Referring to FIG. 5, the ultrasound imaging apparatus 1000 may obtain a trend line 580 indicating a location and a direction of blood flow based on values of blood flow power data obtained at a plurality of points 511 through 575 for each of a plurality of scan lines, i.e., first through seventh scan lines 510 through 570. FIG. 5 shows seven (7) scan lines, i.e., the first through seventh scan lines 510 through 570, for convenience of description, and the number of scan lines (the first through seventh scan lines 510 through 570) is not limited to 7. Furthermore, although FIG. 5 shows that each of the first through seventh scan lines 510 through 570 include five points, the number of points along each of the first through seventh scan lines 510 through 570 is not limited to 5.

In an embodiment, the processor (1400 of FIG. 1) of the ultrasound imaging apparatus 1000 may extract power data having a value equal to or greater than a predetermined threshold from among power data values of blood flow obtained at the points 511 through 575 and identify a point having a value of the extracted power data for each of the first through seventh scan lines 510 through 570. For example, the processor 1400 may extract power data having a value exceeding a threshold among power data values respectively measured at a plurality of points, i.e., first through fifth points 511 through 515, having different depths along the first scan line 510, and identify the second point 512 where the extracted power data is measured from among the first through fifth points 511 through 515. Similarly, the processor 1400 may extract power data having a value exceeding the threshold among power data values respectively measured at a plurality of points, i.e., first through fifth points 521 through 525, having different depths along the second scan line 520, and identify the second point 522 where the extracted power data is measured from among the first through fifth points 521 through 525. In the same manner as described above, the processor 1400 may respectively identify, as points having a value exceeding the threshold, a third point 533 in the third scan line 530, a third point 543 in the fourth scan line 540, a fourth point 554 in the fifth scan line 550, a fifth point 565 in the sixth scan line 560, and a fifth point 575 in the seventh scan line 570.

However, a method of identifying a specific point for each scan line is not limited to the above method. According to an embodiment, the processor 1400 may identify, for each of the first through seventh scan lines 510 through 570, a point having a maximum value of the measured power data from among the points 511 through 575 which are respectively included in the first through seventh scan lines 510 through 570.

In an embodiment, the processor 1400 may obtain the trend line 580 indicating the location and direction of blood flow by connecting the second points 512 and 522, the third points 533 and 543, the fourth point 554, and the fifth points 565 and 575 where the values of extracted power data are measured.

In an embodiment, the ultrasound imaging apparatus 1000 may calculate an angle θ between the obtained trend line 580 and a scan line 590 along which an ultrasound beam is transmitted and correct an angle of a sample volume SV by using the calculated angle θ. According to an embodiment, the processor 1400 of the ultrasound imaging apparatus 1000 may calculate an angle θ formed between the trend line 580 and the scan line 590 where the sample volume SV is located and correct the angle of the sample volume SV by performing an operation of multiplying a velocity of blood flow passing through the sample volume SV by 1/cos θ.

FIG. 6 is a flowchart of a method performed by the ultrasound imaging apparatus 1000, of correcting an angle of a sample volume by using a location and a direction of blood flow, according to an embodiment of the disclosure.

The ultrasound imaging apparatus 1000 extracts power data having a value greater than or equal to a predetermined threshold from among blood flow power data obtained at a plurality of points in an ROI along a plurality of scan lines (operation S610). According to an embodiment, the ultrasound imaging apparatus 1000 may extract, for each of a plurality of scan lines, a point having a maximum value of measured power data from among a plurality of points which are respectively included in the scan lines.

The ultrasound imaging apparatus 1000 obtains a trend line indicating a location and a direction of blood flow by connecting points having values of the extracted power data to one another (operation S620). According to the claimded invention, the ultrasound imaging apparatus 1000 identifies for each of a plurality of scan lines, a point having a power data value greater than or equal to a predetermined threshold from among power data values of blood flow, obtained at a plurality of points in an ROI. According to another embodiment, the ultrasound imaging apparatus 1000 may identify a point having a maximum power data value for each of the scan lines from among power data values of blood flow obtained at the points. The ultrasound imaging apparatus 1000 obtains a trend line indicating a location and a direction of blood flow by connecting the identified points to one another.

The ultrasound imaging apparatus 1000 calculates an angle between the obtained trend line and a scan line along which an ultrasound beam is transmitted (operation S630). The scan line along which the ultrasound beam is transmitted may be a line passing through a position of a sample volume.

The ultrasound imaging apparatus 1000 corrects an angle of a sample volume by using the calculated angle (operation S640). In an embodiment, the ultrasound imaging apparatus 1000 calculates an angle θ formed between a trend line and a scan line where a sample volume is located and corrects an angle of the sample volume by performing an operation of multiplying a velocity of the blood flow passing through the sample volume by 1/cos θ.

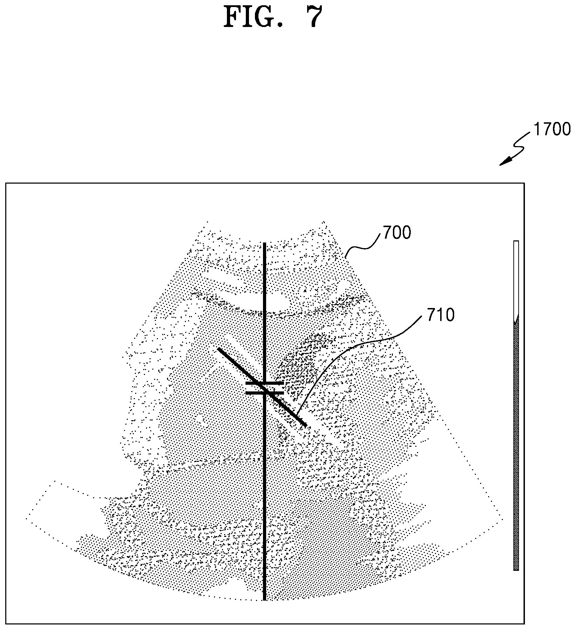

FIG. 7 illustrates an embodiment in which the ultrasound imaging apparatus 1000 of the disclosure displays a trend line for blood flow on a B-mode ultrasound image 700.

Referring to FIG. 7, the display 1700 of the ultrasound imaging apparatus 1000 may display the B-mode ultrasound image 700. The display 1700 may display a trend line 710 by overlaying the trend line on a corresponding position on the B- mode ultrasound image 700. According to an embodiment, the display 1700 may display a GUI representing the trend line 710 as a line or image on the B-mode ultrasound image 700.

FIG. 8A illustrates a frequency spectrum 800 obtained by the ultrasound imaging apparatus 1000 at PRF intervals, according to an embodiment of the disclosure.

Referring to the frequency spectrum 800 shown in FIG. 8A, the ultrasound imaging apparatus 1000 may obtain a plurality of spectral Doppler pulse waves, i.e., first through fifteenth spectral Doppler pulse waves 811 through 825, at a point over time. In an embodiment, the ultrasound imaging apparatus 1000 may store the first through fifteenth spectral Doppler pulse waves 811 through 825 obtained in a time series in the storage (1600 of FIG. 1). An interval between a time point when the first spectral Doppler pulse wave 811 is obtained and a time point when the second spectral Doppler pulse wave 812 is obtained may be equal to an interval of a PRF. According to an embodiment, the ultrasound imaging apparatus 1000 may classify the first through fifteenth spectral Doppler pulse waves 811 through 825 into a plurality of groups by grouping only spectral Doppler pulse waves obtained during a predetermined time period among the first through fifteenth spectral Doppler pulse waves 811 through 825 stored in the storage 1600.

Referring to an embodiment shown in FIG. 8A, the ultrasound imaging apparatus 1000 may classify the first through fifth spectral Doppler pulse waves 811 through 815 as a first group 800-1, the sixth through tenth spectral Doppler pulse waves 816 through 820 as a second group 800-2, and the eleventh through fifteenth spectral Doppler pulse waves 821 through 825 as a third group 800-3. Although FIG. 8A shows that a total number of spectral Doppler pulse waves 811 through 825 is 15 and they are classified into a total of three groups, that is, first, second, and third groups 800-1, 800-2, and 800-3, this is merely an example. The number of spectral Doppler pulse waves (the first through fifteenth spectral Doppler pulse waves 811 through 825) and the number of groups (the first through third groups 800-1, 800-2, and 800-3) are not limited to the ones shown in FIG. 8A.

FIG. 8B is a diagram for describing a method performed by the ultrasound imaging apparatus 1000, of updating trend lines 861 through 863 for blood flow by using power data for spectral Doppler pulse waves obtained based on a predetermined time period, according to an embodiment of the disclosure.

Referring to FIG. 8B, the ultrasound imaging apparatus 1000 may sum up, for each of the first through third groups 800-1, 800-2, and 800-3, power data values of spectral Doppler pulse waves obtained at each of a plurality of points 840-1 through 840-n for each of a plurality of scan lines 831 through 835, and calculate an average of power data values for each of the first through third groups 800-1, 800-2, and 800-3. According to an embodiment, the processor (1400 of FIG. 1) of the ultrasound imaging apparatus 1000 may sum up, for each of the points 840-1 through 840-n, power data values of spectral Doppler pulse waves corresponding to a plurality of pieces of Doppler data 830-1 through 830-5 classified as the first group 800-1 according to a predetermined time period, and calculate an average value of power data for each of the points 840-1 through 840-n by performing an operation of dividing a sum of the power data values by the number of the pieces of Doppler data 830-1 through 830-5. Similarly, the processor 1400 may sum up, for each of the points 840-1 through 840-n, power data values of spectral Doppler pulse waves corresponding to a plurality of pieces of Doppler data 830-6 through 830-10 classified as the second group 800-2, and calculate an average value of power data for each of the points 840-1 through 840-n by performing an operation of dividing a sum of the power data values by the number of the pieces of Doppler data 830-6 through 830-10. The processor 1400 may calculate, for each of the points 840-1 through 840-n, an average value of power data for spectral Doppler pulse waves corresponding to a plurality of pieces of Doppler data 830-11 through 830-15 classified as the third group 800-3.

The ultrasound imaging apparatus 1000 may respectively obtain a plurality of trend lines, i.e., first through third trend lines 861 through 863, indicating a location and a direction of blood flow at a plurality of different time points, i.e., first through third time points t1 through t3, by using the average values of power data calculated for the first through third groups 800-1 through 800-3. According to an embodiment, the processor 1400 may obtain the first trend line 861 indicating a location and a direction of the blood flow at the first time point t1 from Doppler data 850-1 related to the first group 800-1, which represents average values of power data calculated at each of the points 840-1 through 840-n. Because a method of obtaining the first trend line 861 from the Doppler data 850-1 is substantially the same as the method according to the embodiment described with reference to FIGS. 5 and 6, a detailed description thereof will not be repeated below. The processor 1400 may obtain the second trend line 862 indicating a location and a direction of the blood flow at the second time point t2 from Doppler data 850-2 related to the second group 800-2, which represents average values of power data calculated for each of the points 840-1 through 840-n. An interval between the first time point t1 and the second time point t2 may be equal to a predetermined time period Δt. The time period Δt may be set based on a user input. In the same way, the processor 1400 may obtain the third trend line 863 indicating a location and a direction of the blood flow at the third time point t3 from Doppler data 850-3 related to the third group 800-3, which represents average values of power data calculated for each of the points 840-1 through 840-n.

The ultrasound imaging apparatus 1000 may update the first through third trend lines 861 through 863 according to the predetermined time period Δt. According to an embodiment, the processor 1400 may respectively display the first through third trend lines 861 through 863 on the display (1700 of FIG. 1) at the first through third time points t1 through t3.

FIG. 9 is a flowchart of a method by which the ultrasound imaging apparatus 1000 updates a trend line for blood flow by using power data for spectral Doppler pulse waves obtained during a predetermined time period, according to an embodiment of the disclosure.

The ultrasound imaging apparatus 1000 classifies pieces of Doppler data into a plurality of groups by grouping at least one piece of Doppler data obtained during a predetermined time period (operation S910). According to an embodiment, the ultrasound imaging apparatus 1000 may classify a plurality of spectral Doppler pulse waves obtained sequentially at PRF intervals into a plurality of groups based on a predetermined time interval.

The ultrasound imaging apparatus 1000 calculates an average value of power data from at least one piece of Doppler data included in each of the groups (operation S920). According to an embodiment, the ultrasound imaging apparatus 1000 may sum up power data values of spectral Doppler pulse waves obtained at each of a plurality of points in an ROI for each of a plurality of scan lines, and calculate an average of a sum of the power data values for each of the groups.

The ultrasound imaging apparatus 1000 obtains a trend line indicating a location and a direction of blood flow by using the calculated average value (operation S930). According to an embodiment, the ultrasound imaging apparatus 1000 may obtain a trend line by using an average value of power data calculated for each of the groups.

The ultrasound imaging apparatus 1000 updates a location and a direction of the trend line at predetermined time intervals (operation S940). According to an embodiment, the ultrasound imaging apparatus 1000 may display the trend line updated at the predetermined time intervals on the display (1700 of FIG. 1).

In the related art, when the direction of blood flow is changed due to a patient's random motion or breathing, the user is inconvenienced in having to repeatedly correct an angle of a sample volume, which degrades the accuracy of measuring a blood flow velocity.

The ultrasound imaging apparatus 1000 according to the embodiment described with reference to FIGS. 8A, 8B, and 9 may update a trend line indicating a location and a direction of blood flow at predetermined time intervals, thereby allowing the user to identify in real-time the location and direction of blood flow which change due to a patient's breathing or random motion and correct an angle of a sample volume by using the trend line updated in real-time. Accordingly, this may improve the accuracy of measuring a blood flow velocity.

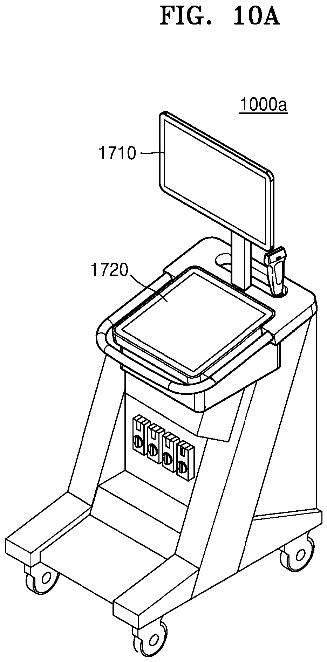

FIG. 10A is diagram illustrating ultrasound diagnosis apparatus according to an exemplary embodiment.

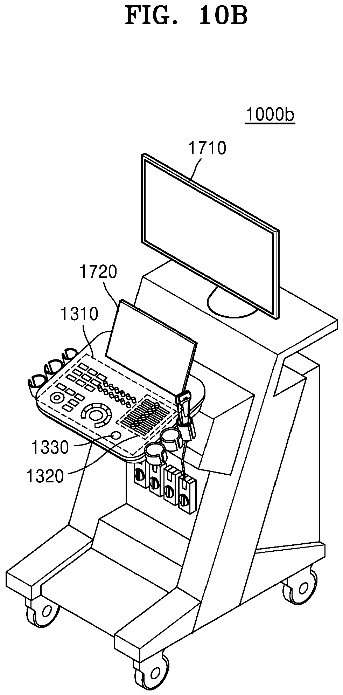

FIG. 10B is diagram illustrating ultrasound imaging apparatus according to an exemplary embodiment.

Referring to FIGS. 10A and 10B, ultrasound imaging apparatuses 1000a and 1000b may include a main display 1710 and a sub-display 1720. At least one among the main display 1710 and the sub-display 1720 may include a touch screen. The main display 1710 and the sub-display 1720 may display ultrasound images and/or various information processed by the ultrasound imaging apparatuses 1000a and 1000b. The main display 1710 and the sub-display 1720 may provide GUIs, thereby receiving user's inputs of data to control the ultrasound imaging apparatuses 1000a and 1000b. For example, the main display 1710 may display an ultrasound image and the sub-display 1720 may display a control panel to control display of the ultrasound image as a GUI. The sub-display 1720 may receive an input of data to control the display of an image through the control panel displayed as a GUI. The ultrasound imaging apparatuses 1000a and 1000b may control the display of the ultrasound image on the main display 1710 by using the input control data.

Referring to FIG. 10B, the ultrasound imaging apparatus 1000b may include a control panel 1310. The control panel 1310 may include buttons, trackballs, jog switches, or knobs, and may receive data to control the ultrasound imaging apparatus 1000b from the user. For example, the control panel 1310 may include a time gain compensation (TGC) button 1320 and a freeze button 1330. The TGC button 1320 is to set a TGC value for each depth of an ultrasound image. Also, when an input of the freeze button 1330 is detected during scanning an ultrasound image, the ultrasound imaging apparatus 1000b may keep displaying a frame image at that time point.

The buttons, trackballs, jog switches, and knobs included in the control panel 1310 may be provided as a GUI to the main display 1710 or the sub-display 1720.

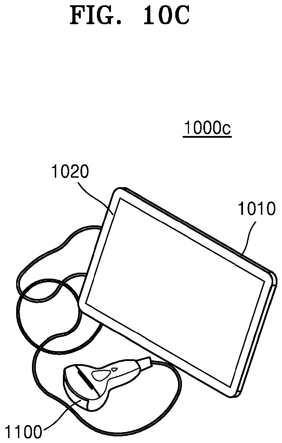

FIG. 10C is diagram illustrating an ultrasound imaging apparatus according to an exemplary embodiment.

Referring to FIG. 10C, the ultrasound imaging apparatus 1000c may include a portable device. An example of a portable ultrasound imaging apparatus may include, for example, smart phones including probes and applications, laptop computers, personal digital assistants (PDAs), or tablet PCs, but an exemplary embodiment is not limited thereto.

The ultrasound imaging apparatus 1000c may include the probe 1100 and a main body 1010. The probe 1100 may be connected to one side of the main body 1010 by wire or wirelessly. The main body 1010 may include a touch screen 1020. The touch screen 1020 may display an ultrasound image, various pieces of information processed by the ultrasound imaging apparatus 1000c, and a GUI.

The embodiments may be implemented as a software program including instructions stored in a computer-readable storage medium.

A computer may refer to a device configured to retrieve an instruction stored in the computer-readable storage medium and to operate, in response to the retrieved instruction, and may include an ultrasound imaging apparatus according to embodiments.

The computer-readable storage medium may be provided in the form of a non-transitory storage medium. In this regard, the term 'non-transitory' means that the storage medium does not include a signal and is tangible, and the term does not distinguish between data that is semi-permanently stored and data that is temporarily stored in the storage medium.

In addition, the ultrasound imaging apparatus or the method of controlling the ultrasound imaging apparatus according to embodiments may be provided in the form of a computer program product. The computer program product may be traded, as a product, between a seller and a buyer.

The computer program product may include a software program and a computer-readable storage medium having stored thereon the software program. For example, the computer program product may include a product (e.g. a downloadable application) in the form of a software program electronically distributed by a manufacturer of the ultrasound imaging apparatus or through an electronic market (e.g., Google ^{™}, Play Store ^{™}, and App Store ^{™}). For such electronic distribution, at least a part of the software program may be stored on the storage medium or may be temporarily generated. In this case, the storage medium may be a storage medium of a server of the manufacturer, a server of the electronic market, or a relay server for temporarily storing the software program.

In a system consisting of a server and a terminal (e.g., the ultrasound imaging apparatus), the computer program product may include a storage medium of the server or a storage medium of the terminal. Alternatively, in a case where a third device (e.g., a smartphone) that communicates with the server or the terminal is present, the computer program product may include a storage medium of the third device. Alternatively, the computer program product may include a software program that is transmitted from the server to the terminal or the third device or that is transmitted from the third device to the terminal.

In this case, one of the server, the terminal, and the third device may execute the computer program product, thereby performing the method according to embodiments. Alternatively, at least two of the server, the terminal, and the third device may execute the computer program product, thereby performing the method according to embodiments in a distributed manner.

For example, the server (e.g., a cloud server, an artificial intelligence (AI) server, or the like) may execute the computer program product stored in the server, and may control the terminal to perform the method according to embodiments, the terminal communicating with the server.

As another example, the third device may execute the computer program product, and may control the terminal to perform the method according to embodiments, the terminal communicating with the third device. In more detail, the third device may remotely control the ultrasound imaging apparatus to emit X-rays to an object, and to generate an image of an inner part of the object, based on detected radiation which passes the object and is detected in an X-ray detector.

As another example, the third device may execute the computer program product, and may directly perform the method according to embodiments, based on at least one value input from an auxiliary device (e.g., a gantry of a CT system). In more detail, the auxiliary device may emit X-rays to an object and may obtain information of radiation which passes the object and is detected in an X-ray detector. The third device may receive an input of signal information about the detected radiation from the auxiliary device, and may generate an image of an inner part of the object, based on the input radiation information.

In a case where the third device executes the computer program product, the third device may download the computer program product from the server, and may execute the downloaded computer program product. Alternatively, the third device may execute the computer program product that is pre-loaded therein, and may perform the method according to the embodiments.

## Claims

1. A method of operating an ultrasonic imaging device (1000) comprising:
transmitting (S210) an ultrasound beam to a region of interest (ROI) including a sample volume in an object;
obtaining Doppler data with respect to a plurality of points in the ROI based on a plurality of scan lines by using a multiline receiving technique;
measuring a velocity of a blood flow passing through the sample volume based on the obtained Doppler data;
obtaining blood flow power data from the Doppler data obtained at the plurality of the points in the ROI;
obtaining (S620, S940) a trend line indicating a location and a direction of the blood flow based on the blood flow power data obtained at the plurality of points in the ROI;
calculating (S630) an angle between the obtained trend line and a scan line where the sample volume is located; and
correcting the measured velocity of the blood flow passing through the sample volume by using the calculated angle,
wherein the obtaining of the trend line comprises:
extracting (S610) power data having a value greater than or equal to a predetermined threshold from among the blood flow power data obtained at the plurality of the points in the ROI;
identifying, for each of the plurality of the scan lines, a point having a value of the extracted power data, from among the plurality of the points in the ROI; and
connecting the points to one another.

2. The method of claim 1, wherein the extracting of the power data comprises extracting, for the each of the plurality of scan lines, power data having a maximum value from among the blood flow power data obtained at the plurality of the points in the ROI.

3. The method of claim 1, wherein the obtaining of the Doppler data comprises:
obtaining a plurality of pieces of Doppler data by repeatedly receiving ultrasound echo signals reflected from the object a plurality of times at intervals of a pulse repetition frequency (PRF);
obtaining the blood flow power data from the plurality of pieces of Doppler data; and
accumulating the obtained blood flow power data over time and storing a plurality of pieces of blood flow power data.

4. The method of claim 3, further comprising:
classifying the plurality of pieces of Doppler data into a plurality of groups by grouping at least one piece of Doppler data obtained during a predetermined time period from among the stored plurality of pieces of blood flow power data according to a predetermined interval;
calculating an average value of power data from the at least one piece of Doppler data included in each of the plurality of groups; and
identifying the location and the direction of the blood flow by using the calculated average value.

5. The method of claim 4, wherein the obtaining of the trend line comprises obtaining the trend line indicating the location and the direction of the blood flow based on the average value calculated for each of the plurality of groups, and
the method further comprises updating the location and the direction of the trend line at predetermined time intervals.

6. An ultrasound imaging apparatus (1000) comprising:
an ultrasound transceiver (1200) configured to transmit an ultrasound beam to a region of interest (ROI) including a sample volume in an object and receive ultrasound echo signals reflected from the ROI along a plurality of scan lines by using a multiline receiving technique;
a storage (1600) storing the received ultrasound echo signals for each of the plurality of echo signals;
a memory (1500) storing at least one instruction; and
a processor (1400) configured to execute the at least one instruction stored in the memory to:
obtain Doppler data with respect to a plurality of points in the ROI for each of the plurality of scan lines based on the ultrasound echo signals stored in the storage,
measure a velocity of a blood flow passing through the sample volume based on the obtained Doppler data,
obtain blood flow power data from the Doppler data obtained at the plurality of the points in the ROI,
obtain a trend line indicating a location and a direction of a blood flow based on the blood flow power data obtained at the plurality of points in the ROI,
calculate an angle between the obtained trend line and a scan line where the sample volume is located and
correct the measured velocity of the blood flow passing through the sample volume by using the calculated angle,
wherein obtaining the trend line comprises:
extracting power data having a value greater than or equal to a predetermined threshold from among the blood flow power data obtained at the plurality of the points in the ROI;
identifying, for each of the plurality of the scan lines, a point having a value of the extracted power data, from among the plurality of the points in the ROI; and
connecting the points to one another.

7. The ultrasound imaging apparatus (1000) of claim 6, wherein the processor is further configured to execute the at least one instruction to extract, for each of the plurality of scan lines, power data having a maximum value from among the blood flow power data.

8. The ultrasound imaging apparatus (1000) of claim 6, wherein the ultrasound transceiver (1200) is further configured to obtain a plurality of pieces of Doppler data by repeatedly receiving ultrasound echo signals reflected from the object a plurality of times at intervals of a pulse repetition frequency (PRF), and
wherein the processor (1400) is further configured to execute the at least one instruction to:
obtain the blood flow power data from the plurality of pieces of Doppler data;
accumulate the obtained blood flow power data over time and store a plurality of pieces of blood flow power data; and
calculate an average value of the plurality of pieces of blood flow power data stored in the storage at each of a plurality of points in the ROI.

9. The ultrasound imaging apparatus (1000) of claim 8, wherein the processor (1400) is further configured to execute the at least one instruction to:
classify the plurality of pieces of Doppler data into a plurality of groups each including at least one piece of Doppler data obtained during a predetermined time period from among the plurality of pieces of blood flow power data stored in the storage according to a predetermined time interval;
calculate an average value of power data from the at least one piece of Doppler data included in each of the plurality of groups; and
identify the location and the direction of the blood flow by using the calculated average value.

10. The ultrasound imaging apparatus (1000) of claim 9, wherein the processor (1400) is further configured to execute the at least one instruction to:
obtain the trend line indicating the location and the direction of the blood flow based on the average value calculated for each of the plurality of groups; and
update the location and the direction of the trend line at predetermined time intervals.

11. A non-transitory computer-readable recording medium having recorded thereon a program for performing the method of any of claims 1-5 on a computer.

## Patentansprüche

1. Verfahren zum Betrieb einer Ultraschallbildgebungsvorrichtung (1000), umfassend:
Senden (S210) eines Ultraschallstrahls zu einer ein Probenvolumen enthaltenden Region von Interesse (ROI, Region Of Interest) in einem Objekt;
Erhalten von Doppler-Daten in Bezug auf eine Vielzahl von Punkten in der ROI basierend auf einer Vielzahl von Scanlinien unter Verwendung einer Mehrlinien-Empfangstechnik;
Messen einer Geschwindigkeit eines Blutflusses, der durch das Probenvolumen fließt, basierend auf den erhaltenen Doppler-Daten;
Erhalten von Blutfluss-Leistungsdaten aus den an der Vielzahl von Punkten in der ROI erhaltenen Doppler-Daten;
Erhalten (S620, S940) einer Trendlinie, die Ort und Richtung des Blutflusses anzeigt, und zwar basierend auf den an der Vielzahl von Punkten in der ROI erhaltenen Blutfluss-Leistungsdaten;
Berechnen (S630) eines Winkels zwischen der erhaltenen Trendlinie und einer Scanlinie, wo sich das Probenvolumen befindet; und
Korrigieren der gemessenen Geschwindigkeit des Blutflusses, der durch das Probenvolumen fließt, unter Verwendung des berechneten Winkels,
wobei das Erhalten der Trendlinie Folgendes umfasst:
Extrahieren (S610) von Leistungsdaten aus den an der Vielzahl von Punkten in der ROI erhaltenen Blutfluss-Leistungsdaten, die einen Wert aufweisen, der größer oder gleich einem vorbestimmten Schwellenwert ist;
Identifizieren eines Punktes aus der Vielzahl von Punkten in der ROI, der einen Wert der extrahierten Leistungsdaten aufweist, für jede aus der Vielzahl von Scanlinien; und
Verbinden der Punkte miteinander.

2. Verfahren nach Anspruch 1, wobei das Extrahieren der Leistungsdaten Folgendes umfasst: Extrahieren von Leistungsdaten aus den an der Vielzahl von Punkten in der ROI erhaltenen Blutfluss-Leistungsdaten, die einen Maximalwert aufweisen, für jede aus der Vielzahl von Scanlinien.

3. Verfahren nach Anspruch 1, wobei das Erhalten der Doppler-Daten Folgendes umfasst:
Erhalten einer Vielzahl von Doppler-Datenelementen durch wiederholtes Empfangen von Ultraschall-Echosignalen, die von dem Objekt mehrmals in Intervallen einer Pulswiederholungsfrequenz (PRF, Pulse Repetition Frequency) reflektiert werden;
Erhalten der Blutfluss-Leistungsdaten aus der Vielzahl von Doppler-Datenelementen; und
Akkumulieren der erhaltenen Blutfluss-Leistungsdaten im Zeitverlauf und Speichern einer Vielzahl von Blutfluss-Leistungsdatenelementen.

4. Verfahren nach Anspruch 3, ferner umfassend:
Klassifizieren der Vielzahl von Doppler-Datenelementen in eine Vielzahl von Gruppen durch Gruppieren zumindest eines Doppler-Datenelements, das während einer vorbestimmten Zeitdauer aus der Vielzahl von gespeicherten Blutfluss-Leistungsdatenelemente erhalten wurde, gemäß einem vorbestimmten Intervall;
Berechnen eines Durchschnittswerts von Leistungsdaten aus dem zumindest einen Doppler-Datenelement, das in jeder der Vielzahl von Gruppen enthalten ist; und
Identifizieren von Ort und Richtung des Blutflusses unter Verwendung des berechneten Durchschnittswerts.

5. Verfahren nach Anspruch 4, wobei das Erhalten der Trendlinie das Erhalten der Trendlinie umfasst, die Ort und Richtung des Blutflusses anzeigt, und zwar basierend auf dem für jede aus der Vielzahl von Gruppen berechneten Durchschnittswert,
wobei das Verfahren ferner das Aktualisieren von Ort und Richtung der Trendlinie in vorbestimmten Zeitintervallen umfasst.

6. Ultraschallbildgebungsvorrichtung (1000), die Folgendes umfasst:
einen Ultraschalltransceiver (1200), der so konfiguriert ist, dass er einen Ultraschallstrahl zu einer ein Probenvolumen enthaltenden Region von Interesse (ROI, Region Of Interest) in einem Objekt sendet, und von der ROI reflektierte Ultraschallechosignale entlang einer Vielzahl von Scanlinien unter Verwendung einer Mehrlinien-Empfangstechnik empfängt;
einen Speicher (1600), in dem die empfangenen Ultraschallechosignale für jedes aus der Vielzahl von Echosignalen gespeichert werden;
einen Arbeitsspeicher (1500), in dem zumindest eine Anweisung gespeichert ist; und
einen Prozessor (1400), der so konfiguriert ist, dass er die zumindest eine in dem Arbeitsspeicher gespeicherte Anweisung zu Folgendem ausführt:
Erhalten von Doppler-Daten in Bezug auf eine Vielzahl von Punkten in der ROI für jede aus der Vielzahl von Scanlinien basierend auf den in dem Speicher gespeicherten Ultraschallechosignalen,
Messen einer Geschwindigkeit eines Blutflusses, der durch das Probenvolumen fließt, basierend auf den erhaltenen Doppler-Daten,
Erhalten von Blutfluss-Leistungsdaten aus den an der Vielzahl von Punkten in der ROI erhaltenen Doppler-Daten,
Erhalten einer Trendlinie, die Ort und Richtung eines Blutflusses anzeigt, und zwar basierend auf den an der Vielzahl von Punkten in der ROI erhaltenen Blutfluss-Leistungsdaten,
Berechnen eines Winkels zwischen der erhaltenen Trendlinie und einer Scanlinie, wo sich das Probenvolumen befindet, und
Korrigieren der gemessenen Geschwindigkeit des Blutflusses, der durch das Probenvolumen fließt, unter Verwendung des berechneten Winkels,
wobei das Erhalten der Trendlinie Folgendes umfasst:
Extrahieren von Leistungsdaten, die einen Wert aufweisen, der größer oder gleich einem vorbestimmten Schwellenwert ist, aus den an der Vielzahl von Punkten in der ROI erhaltenen Blutfluss-Leistungsdaten;
Identifizieren eines Punktes aus der Vielzahl von Punkten in der ROI, der einen Wert der extrahierten Leistungsdaten aufweist, für jede der Vielzahl von Scanlinien; und
Verbinden der Punkte miteinander.

7. Ultraschallbildgebungsvorrichtung (1000) nach Anspruch 6, wobei der Prozessor ferner so konfiguriert ist, dass er die zumindest eine Anweisung ausführt, um für jede aus der Vielzahl von Scanlinien Leistungsdaten zu extrahieren, die einen Maximalwert unter den Blutfluss-Leistungsdaten aufweisen.

8. Ultraschallbildgebungsvorrichtung (1000) nach Anspruch 6, wobei der Ultraschalltransceiver (1200) ferner zum Erhalten einer Vielzahl von Doppler-Datenelementen durch wiederholtes Empfangen von Ultraschall-Echosignalen, die von dem Objekt mehrmals in Intervallen einer Pulswiederholungsfrequenz (PRF, Pulse Repetition Frequency) reflektiert werden, konfiguriert ist, und
wobei der Prozessor (1400) ferner so konfiguriert ist, dass er die mindestens eine Anweisung zu Folgendem ausführt:
Erhalten der Blutfluss-Leistungsdaten aus der Vielzahl von Doppler-Datenelementen; und
Akkumulieren der erhaltenen Blutfluss-Leistungsdaten im Zeitverlauf und Speichern einer Vielzahl von Blutfluss-Leistungsdaten; und
Berechnen eines Durchschnittswertes aus der Vielzahl von in dem Speicher gespeicherten Blutfluss-Leistungsdatenelementen an jedem von einer Vielzahl von Punkten in der ROI.

9. Ultraschallbildgebungsvorrichtung (1000) nach Anspruch 8, wobei der Prozessor (1400) ferner so konfiguriert ist, dass er die mindestens eine Anweisung zu Folgendem ausführt:
Klassifizieren der Vielzahl von Doppler-Datenelementen in eine Vielzahl von Gruppen, die zumindest ein Doppler-Datenelement enthalten, das während einer vorbestimmten Zeitdauer aus der Vielzahl von in dem Speicher gespeicherten Blutfluss-Leistungsdatenelementen erhalten wurde, gemäß einem vorbestimmten Zeitintervall;
Berechnen eines Durchschnittswerts von Leistungsdaten aus dem zumindest einen Doppler-Datenelement, das in jeder der Vielzahl von Gruppen enthalten ist; und
Identifizieren von Ort und Richtung des Blutflusses unter Verwendung des berechneten Durchschnittswerts.

10. Ultraschallbildgebungsvorrichtung (1000) nach Anspruch 9, wobei der Prozessor (1400) ferner so konfiguriert ist, dass er die mindestens eine Anweisung zu Folgendem ausführt:
Erhalten der Trendlinie, die Ort und Richtung des Blutflusses anzeigt, und zwar basierend auf dem für jede aus der Vielzahl von Gruppen berechneten Durchschnittswert; und
Aktualisieren von Ort und Richtung der Trendlinie in vorbestimmten Zeitintervallen.

11. Übergangsloses computerlesbares Aufzeichnungsmedium, auf dem ein Programm zur Durchführung des Verfahrens nach einem der Ansprüche 1 bis 5 auf einem Computer gespeichert ist.

## Revendications

1. Procédé d'exploitation d'un dispositif d'échographie (1000) comprenant les opérations consistant à :
émettre (S210) un faisceau d'ultrasons vers une région d'intérêt (ROI, region of interest), qui comprend un volume échantillon, dans un objet,
obtenir des données Doppler par rapport à une pluralité de points présents dans la ROI compte tenu d'une pluralité de lignes de balayage au moyen d'une technique de réception multiligne,
mesurer la vitesse d'un flux sanguin traversant le volume échantillon compte tenu des données Doppler obtenues,
obtenir des données de puissance de flux sanguin à partir des données Doppler obtenues au niveau de la pluralité de points présents dans la ROI,
obtenir (S620, S940) une ligne de tendance indiquant l'emplacement et la direction du flux sanguin compte tenu des données de puissance de flux sanguin obtenues au niveau de la pluralité de points présents dans la ROI,
calculer (S630) un angle entre la ligne de tendance obtenue et une ligne de balayage où se trouve le volume échantillon, et
corriger la vitesse mesurée du flux sanguin traversant le volume échantillon au moyen de l'angle calculé ;
ladite obtention de la ligne de tendance comprenant les opérations consistant à :
extraire (S610) des données de puissance ayant une valeur supérieure ou égale à un seuil prédéterminé parmi les données de puissance de flux sanguin obtenues au niveau de la pluralité de points présents dans la ROI,
identifier, pour chaque ligne de la pluralité de lignes de balayage, un point dont la valeur est celle des données de puissance extraites, parmi la pluralité des points présents dans la ROI, et
relier les points entre eux.

2. Procédé selon la revendication 1, dans lequel l'extraction des données de puissance comprend l'extraction, pour chaque ligne de la pluralité de lignes de balayage, de données de puissance ayant une valeur maximale parmi les données de puissance de flux sanguin obtenues au niveau de la pluralité de points présents dans la ROI.

3. Procédé selon la revendication 1, dans lequel l'obtention des données Doppler comprend les opérations consistant à :
obtenir une pluralité d'éléments de données Doppler par réception répétée de signaux d'écho ultrasonores, réfléchis par l'objet une pluralité de fois, à des intervalles d'une fréquence de répétition d'impulsions (PRF, pulse repetition frequency),
obtenir les données de puissance de flux sanguin à partir de la pluralité d'éléments de données Doppler, et
accumuler dans le temps les données de puissance de flux sanguin obtenues et stocker une pluralité d'éléments de données de puissance de flux sanguin.

4. Procédé selon la revendication 3, comprenant en outre les opérations consistant à :
classer la pluralité d'éléments de données Doppler en une pluralité de groupes par groupement d'au moins un élément de données Doppler obtenu pendant une période prédéterminée parmi la pluralité stockée d'éléments de données de puissance de flux sanguin selon un intervalle prédéterminé,
calculer une valeur moyenne de données de puissance à partir de l'au moins un élément de données Doppler présent dans chaque groupe de la pluralité de groupes, et
identifier l'emplacement et la direction du flux sanguin au moyen de la valeur moyenne calculée.

5. Procédé selon la revendication 4, dans lequel l'obtention de la ligne de tendance comprend l'obtention de la ligne de tendance indiquant l'emplacement et la direction du flux sanguin compte tenu de la valeur moyenne calculée pour chaque groupe de la pluralité de groupes, et
le procédé comprend en outre l'actualisation de l'emplacement et de la direction de la ligne de tendance à des intervalles de temps prédéterminés.

6. Appareil d'échographie (1000) comprenant :
un émetteur-récepteur à ultrasons (1200) conçu pour émettre un faisceau d'ultrasons vers une région d'intérêt (ROI, region of interest), qui comprend un volume échantillon, dans un objet et pour recevoir des signaux d'écho ultrasonores, réfléchis par la ROI, le long d'une pluralité de lignes de balayage au moyen d'une technique de réception multiligne,
un dispositif de stockage (1600) dans lequel sont stockés les signaux d'écho ultrasonores reçus pour chaque signal de la pluralité de signaux d'écho,
une mémoire (1500) dans laquelle est stockée au moins une instruction, et
un processeur (1400) conçu pour exécuter au moins une instruction stockée dans la mémoire pour :
obtenir des données Doppler par rapport à une pluralité de points présents dans la ROI pour chaque ligne de la pluralité de lignes de balayage compte tenu des signaux d'écho ultrasonores stockés dans le dispositif de stockage,
mesurer la vitesse d'un flux sanguin traversant le volume échantillon compte tenu des données Doppler obtenues,
obtenir des données de puissance de flux sanguin à partir des données Doppler obtenues au niveau de la pluralité de points présents dans la ROI,
obtenir une ligne de tendance indiquant l'emplacement et la direction d'un flux sanguin compte tenu des données de puissance de flux sanguin obtenues au niveau de la pluralité de points présents dans la ROI,
calculer un angle entre la ligne de tendance obtenue et une ligne de balayage où se situe le volume échantillon, et
corriger la vitesse mesurée du flux sanguin traversant le volume échantillon au moyen de l'angle calculé ;
ladite obtention de la ligne de tendance comprenant les opérations consistant à :
extraire des données de puissance ayant une valeur supérieure ou égale à un seuil prédéterminé parmi les données de puissance de flux sanguin obtenues au niveau de la pluralité de points présents dans la ROI,
identifier, pour chaque ligne de la pluralité de lignes de balayage, un point dont la valeur est celle des données de puissance extraites, parmi la pluralité des points présents dans la ROI, et
relier les points entre eux.

7. Appareil d'échographie (1000) selon la revendication 6, dans lequel le processeur est en outre conçu pour exécuter l'au moins une instruction pour extraire, pour chaque ligne de la pluralité de lignes de balayage, des données de puissance ayant une valeur maximale parmi les données de puissance de flux sanguin.

8. Appareil d'échographie (1000) selon la revendication 6, dans lequel l'émetteur-récepteur à ultrasons (1200) est en outre conçu pour obtenir une pluralité d'éléments de données Doppler par réception répétée de signaux d'écho ultrasonores, réfléchis par l'objet une pluralité de fois, à des intervalles d'une fréquence de répétition d'impulsions (PRF, pulse repetition frequency), et
ledit processeur (1400) étant en outre conçu pour exécuter l'au moins une instruction pour :
obtenir les données de puissance de flux sanguin à partir de la pluralité d'éléments de données Doppler,
accumuler dans le temps les données de puissance de flux sanguin obtenues et stocker une pluralité de données de puissance de flux sanguin, et
calculer une valeur moyenne de la pluralité d'éléments de données de puissance de flux sanguin stockées dans le dispositif de stockage à chaque point d'une pluralité de points présents dans la ROI.

9. Appareil d'échographie (1000) selon la revendication 8, dans lequel le processeur (1400) est en outre conçu pour exécuter l'au moins une instruction pour :
classer la pluralité d'éléments de données Doppler en une pluralité de groupes comprenant chacun au moins un élément de données Doppler obtenu pendant une période prédéterminée parmi la pluralité d'éléments de données de puissance de flux sanguin stockés dans le dispositif de stockage selon un intervalle de temps prédéterminé,
calculer une valeur moyenne de données de puissance à partir de l'au moins un élément de donnée Doppler présent dans chaque groupe de la pluralité de groupes, et
identifier l'emplacement et la direction du flux sanguin au moyen de la valeur moyenne calculée.

10. Appareil d'échographie (1000) selon la revendication 9, dans lequel le processeur (1400) est en outre conçu pour exécuter l'au moins une instruction pour :
obtenir la ligne de tendance indiquant l'emplacement et la direction du flux sanguin compte tenu de la valeur moyenne calculée pour chaque groupe de la pluralité de groupes, et
actualiser l'emplacement et la direction de la ligne de tendance à des intervalles de temps prédéterminés.

11. Support d'enregistrement non transitoire lisible par ordinateur sur lequel est enregistré un programme permettant d'exécuter le procédé selon l'une quelconque des revendications 1 à 5 sur un ordinateur.
